# EUROPEAN PATENT SPECIFICATION

(11) **EP 3 700 432 B1**
(45) Date of publication and mention of the grant of the patent: **12.02.2025**
(21) Application number: 18807438.9
(22) Date of filing: 10.10.2018
(51) Int. Cl.: A61B 17/00

(54) **IMPLANTABLE ATRIAL SEPTAL DEFECT OCCLUSION DEVICE WITH WOVEN CENTRAL SECTION ON LEFT ATRIAL FLANGE**
IMPLANTIERBARE VORRICHTUNG FÜR DEN VERSCHLUSS EINES VORHOFSEPTUMDEFEKTS MIT GEWEBTEM MITTELABSCHNITT AUF DEM LINKEN VORHOFFLANSCH
DISPOSITIF D'OCCLUSION DE COMMUNICATION INTER-AURICULAIRE IMPLANTABLE AYANT UNE SECTION CENTRALE TISSÉE SUR UNE BRIDE AURICULAIRE GAUCHE

(30) Priority: 13.10.2017 IN 201741036490
(43) Date of publication of application: 02.09.2020
(73) Proprietor: Sree Chitra Tirunal Institute for Medical Sciences & Technology, Thiruvananthapuram-695012, Kerala (IN)
(72) Inventor: SREEDHARAN, Sujesh, Triruvananthapuram- 695012, Kerala (IN); JERARD, Jijo, Triruvananthapuram- 695012, Kerala (IN); VIJAYAN, Liji Geetha, Triruvananthapuram- 695012, Kerala (IN); SASIDHARAN, Bijulal, Triruvananthapuram- 695012, Kerala (IN)
(74) Representative: Stöckeler, Ferdinand
(86) International application number: PCT/IN2018/050642
(87) International publication number: WO 2019/073480

(56) References cited:
- WO-A1-2008/125689
- WO-A1-2016/087504
- CN-Y- 2 524 710
- US-A1- 2006 224 183

## Description

### FIELD OF INVENTION

The present invention relates to implantable atrial septal defect occlusion devices employed for transcatheter closure of atrial septal defect.

### BACKGROUND OF THE INVENTION

Atrial septal defects are a type of congenital heart disease where the atrial wall or septum separating the right and left atria are not completely formed. To treat this condition, either it is surgically closed or can be closed percutaneously using closure devices. Closure devices comprise of a two part system where the device has a frame to support the device in place and a scaffold made of a thrombogenic substance to close the defect.

The first clinical devices had stainless steel frames supporting polyester patches to close the defect. Many patterns of closing the defects were available with different closure material and different retaining structures. Currently, shape memory alloy (SMA) and super-elastic materials are widely used in the frame. Use of Nitinol wire braided frame to form the device has wide acceptance because of its ease of delivery and better fixation.

WO 2008/125 689 discloses an occluder. US 2006 / 0 224 183 A1 and CN 2 524 710 Y each disclose an implantable device. WO 2016 / 087 504 A1 discloses a medical closure device.

Some of the prior art in this field detail about working on a plurality of metal strands heat treated to attain a shape and sustain it after it is collapsed into a catheter and later deployed (US5846261). Patent US5725552 describes the method of forming a medical device from a plurality of metal wires by shaping and heat treatment using a mould. Such a device is said to be collapsible for passage through a catheter for deployment in a channel of a patient's body. Patent US6362339B1 describes the method where a plurality of metal wires is heat treated to conform to the surface of a molding element to attain a predetermined shape which is a two lobed structure. Different techniques of forming the metal fabric are described in patent US 9179899 B2 where it is formed by knitting wires and the structure is described as having proximal and distal portions which are orderly deployed in a particular unique way to close a cavity in the human body. Patents (US20070225760A1, DE102005053906A1) which claim a closed left atrial (LA) side use different braiding techniques to obtain a closed wall like formation on the LA side of the devices.

Braided designs of the wires are used to form a two lobed structure which while deploying, open on two sides of the defect closing it. The devices commonly have two lobes connected by a neck. The braids are held together by hubs on both proximal and distal ends with the distal end having suitable releasing mechanism to deploy the device. These hubs might cause a slight obstruction to normal blood flow. Also these hubs are the regions where it takes more time for endothelium formation. Device migration and atrial roof erosion are two concerns with the present designs. There is a need for improvement of the device in terms of its structural stability and fixation and the present invention addresses the above need.

### OBJECTS OF THE INVENTION

Therefore, it is an object of the invention to propose an implantable atrial septal defect occlusion device made of braided metallic wires with a woven central section on its left atrial flange.

Another object of the invention is to propose an implantable atrial septal defect occlusion device with woven central section on left atrial flange which occupies less volume inside the left atrium by reducing the size of the hub or avoiding it altogether.

A still another object of the invention is to propose an implantable atrial septal defect occlusion device with woven central section on left atrial flange, which is capable of providing structural stability of the LA flange by using the novel woven central section.

A further object of the invention is to propose an implantable atrial septal defect occlusion device with woven central section on left atrial flange, which is able to provide better clasping strength on to the septal wall by introducing a ridge on the periphery of either the LA flange or the right atrial (RA) flange or both flanges thus increasing the migration resistance.

A still further object of the invention is to propose an implantable atrial septal defect occlusion device with woven central section on left atrial flange, which is capable of reducing atrial wall erosion, especially at the atrial roof, by employing a rounded ridge on the periphery of the flanges.

### SUMMARY OF INVENTION

The scope of protection is defined by the appended claims.

A medical device which is the embodiment of the present invention for the closure of cardiac septal defects such as atrial septal defect is described here. The device is a braided collapsible and expandable structure with two discs (flanges) and a connecting neck in-between them and further described by claim 1. The neck fits into the septal defect with the flanges clamping on to the sides of the defect wall supporting it and at the same time maintaining a low profile to prevent any obstruction to normal blood flow. The low profile is maintained by use of a novel woven central section which also provides structural stability to the hub-less flange. The device may comprise a metal mesh, such as a super-elastic metal mesh, shaped into the device with the thrombogenic material inside for closure of the defect. The ridges enhance the fixation on the septal wall and provide a softer edge to reduce chances of atrial roof erosion.

This device scaffolds the remaining septal portion and helps in completing the atrial septum, thus preventing the inter-atrial blood flow. The device occupies minimal volume in the atria and provides minimum obstruction to blood flow in the heart chamber. The device is placed into the defect using a catheter and deployed on unsheathing from the catheter. The present invention improves the prior art in terms of minimising atrial volume occupied and providing better scaffolding by use of a woven central section on the left atrial disc and a ridge on its periphery; and reducing possible atrial wall erosion.

### BRIEF DESCRIPTION OF THE ACCOMPANYING DRAWINGS

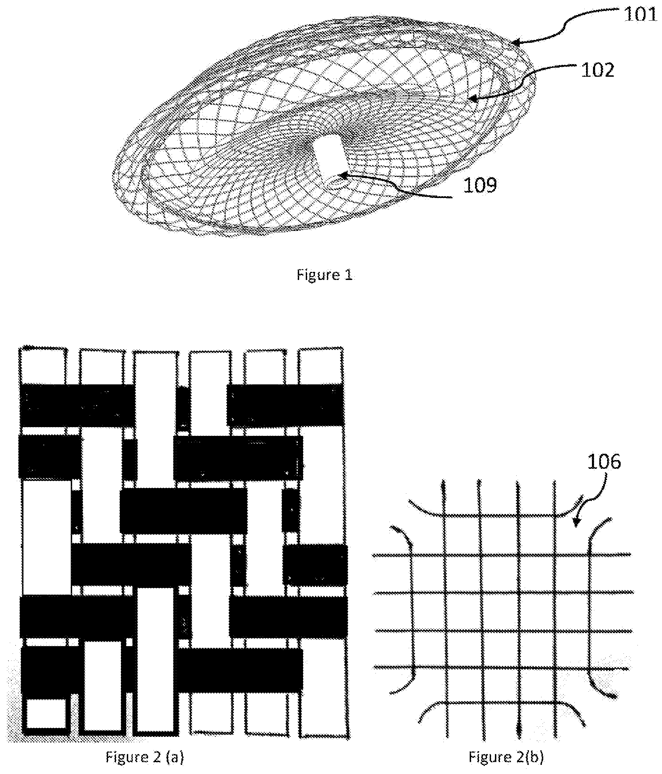

Fig 1 shows an embodiment of the invention showing RA side and a portion of LA side with the free ends of the wires inserted into a hub on the RA side.
Fig 2(a) shows the representation of the twill weave which is done using the first fraction of the wires forming the device.
Fig 2(b) shows arrangement of wires in the weave with corners opened.

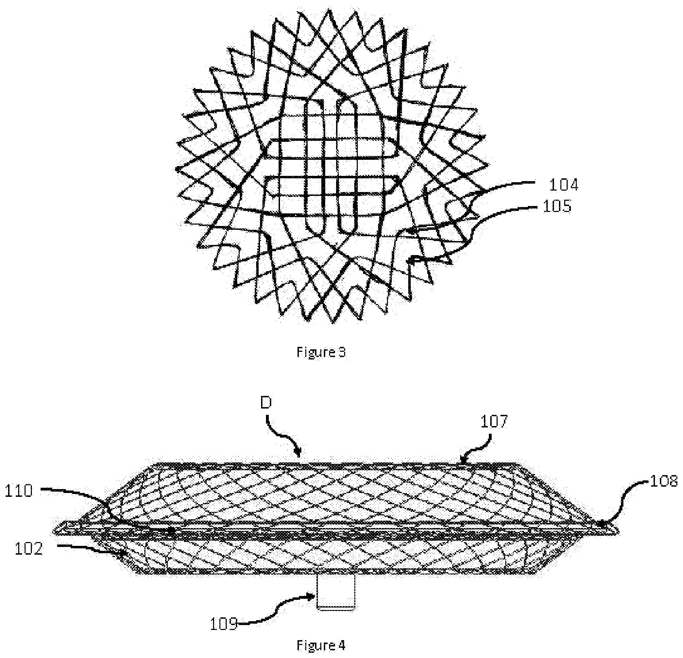

Fig 3 shows the pictorial representation of the LA side central section with the braids shown after the introduction of all the wires in three stages.
Fig 4 shows the side view of the device showing the LA and RA sides and the neck region connecting the two discs.
Fig 5 shows the isometric view of the invention showing ridge on the LA flange.

### DETAILED DESCRIPTION OF A PREFERRED EMBODIMENT OF THE INVENTION

A device for occluding atrial septal defect is disclosed here.

A transcatheter device for use as an atrial septal defect occluder has two discs, one a hub-less disc incorporating a woven central section on the left atrial side and the other disc on the right atrial side with a connecting neck braided from wires and the device has thrombogenic material in either discs and the said neck with a ridge along the periphery of either the left atrial disc or the right atrial disc or both discs.

Wires are braided and shaped into a two lobed structure and heat set into the required shape. Metal alloys such as Nitinol can be used to braid the device and shaped as is well known in the art. The braided structure has a closed end which forms the LA flange (101) of the device thus avoiding a hub and ensuring that minimum volume is occupied in the left atrium. The device having no hub and a flat central section on the left atrial disc occupying lesser volume inside the left atrium and results in improved endothelialization.

The closing of the LA flange has been achieved in the prior art either by (i) crisscrossing all the wires across the centre or (ii) crisscrossing only a part while looping back the remaining part in a particular pattern.

The method discussed here uses a novel combination of weaving the wires to form a mat like central section with one part of the wires, and then introducing the other parts in either a single stage or multiple stages, introducing them as loops. Looped wires are introduced in one or more stages, with each stage interspaced with one or more braids, and braided together with the wires from the woven centre and earlier stages to form the device. The next stages are introduced after one or more braids of the existing wires and finally all strands are braided together. The braid is a regular braid whereas the weave could be regular or a twill weave, (105). The final braid could incorporate either a regular braid or a diamond braid.

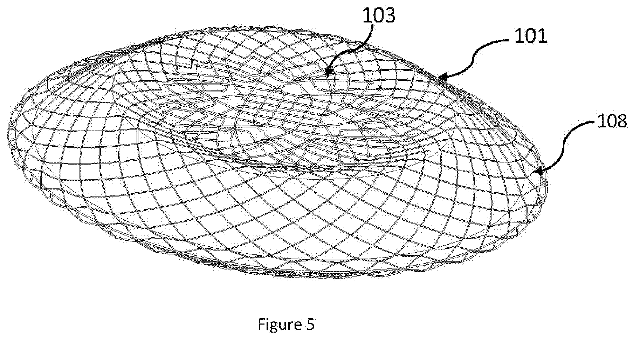

The woven wires are introduced onto a mandrel which holds the weave in place and the open ends are braided. Then the next stages of wires are added as loops, (104) after the formation of the braid between the woven wires. The newly introduced wires are braided with the existing braids forming a single braid; after two sets of braiding the final set of wires are added and braided on a suitable mandrel with closed end as visible in Figure 5.

The second (104) and third (105) stages of wires are introduced onto the stubs on the mandrel which initially holds them in place before being braided onto the mandrel.

The wire mesh, (103) is formed with the weave mat (106) on the top and the wires introduced in subsequent stages (104 & 105), all getting braided together. The wire mesh is shaped into the required device shape of two flange structure with a connecting neck (110).

The weave design on the end results in the formation of a flat (107) end which helps in reducing the volume occupied by the device in the atrium. The hub-less flange can facilitate a continuous endothelium formation on top of the device. The metal mesh will be shaped into the twin-disc frame form and heat set; a material with thrombogenic properties, such as non-woven thin fabrics, is inserted into the twin discs and the neck region to induce closure of the atrial septal defect when the device is deployed in it.

The distal end where the wire ends (109) are combined together in a hub incorporating the release mechanism.

Further, a ridge (108) can be introduced on the periphery of either the LA flange or the RA flange, or both flanges to improve the structural stability of the device and provides enhanced clasping force onto the septum reducing chances of migration. The peripheral ridge gives a softer edge for the device reducing chances of atrial roof erosion.

## Claims

1. An implantable atrial septal defect occlusion device (D) comprising:
a braided collapsible and expandable structure with a proximal disc (102), a distal disc (101) and a connecting neck in-between said discs (101, 102), wherein each of said discs (101, 102) defines a respective periphery; and
a ridge (108) disposed on the periphery of at least one of the discs (101, 102);
wherein the distal disc (101) is a hub-less disc and is configured for the left atrial side whereas the proximal disc (102) is configured for the right atrial side; and wherein said discs (101, 102) comprise a thrombogenic material
**characterized in that** the distal, hub-less disc (101) incorporates a woven central section (106) and said device (D) comprises non-woven wires (109) configured in one or more stages, with each stage interspaced with one or more braids and braided together with the wires from the woven central section (106) and earlier stages to form the device (D).

2. The device (D) as claimed in claim 1, wherein a weave of the central section (106) is a plain weave or a twill weave.

3. The device (D) as claimed in claim 1, wherein a distal end where wire ends of the non-woven wires (109) meet are combined together in a hub incorporating a release mechanism.

## Patentansprüche

1. Eine implantierbare Atriumseptumdefekt-Okklusionsvorrichtung (D), die folgende Merkmale aufweist
eine geflochtene zusammenklappbar und erweiterbar Struktur mit einer proximalen Scheibe (102), einer distalen Scheibe (101) und einem Verbindungshals zwischen den Scheiben (101, 102), wobei jede der Scheiben (101, 102) einen jeweiligen Umfang definiert; und
einen Grat (108), der an dem Umfang zumindest einer der Scheiben (101, 102) angeordnet ist;
wobei die distale Scheibe (101) eine nabenlose Scheibe ist und für die linke Atriumseite konfiguriert ist, wohingegen die proximale Scheibe (102) für die rechte Atriumseite konfiguriert ist; und wobei die Scheiben (101, 102) ein thrombogenes Material aufweisen,
**dadurch gekennzeichnet, dass** die distale nabenlose Scheibe (101) einen gewebten zentralen Abschnitt (106) aufweist und die Vorrichtung (D) nicht-gewebte Drähte (109) aufweist, die in einer oder mehreren Stufen konfiguriert sind, wobei jede Stufe mit einem oder mehreren Geflechten beabstandet ist und mit den Drähten aus dem gewebten zentralen Abschnitt (106) und früheren Stufen zusammengeflochten ist, um die Vorrichtung (D) zu bilden.

2. Die Vorrichtung (D) gemäß Anspruch 1, wobei eine Bindung des zentralen Abschnitts (106) eine Leinwandbindung oder eine Köperbindung ist.

3. Die Vorrichtung (D) gemäß Anspruch 1, wobei distale Enden, an dem Drahtenden der nicht-gewebten Drähte (109) zusammentreffen, in einer Nabe kombiniert sind, die einen Freigabemechanismus aufweist.

## Revendications

1. Dispositif d'occlusion de communication inter-auriculaire implantable (D) comprenant :
une structure tressée compressible et expansible avec un disque proximal (102), un disque distal (101) et un col de liaison entre lesdits disques (101, 102), dans lequel chacun desdits disques (101, 102) définit une périphérie respective ; et
une arête (108) disposée sur la périphérie d'au moins un des disques (101, 102) ;
dans lequel le disque distal (101) est un disque sans moyeu et est configuré pour le côté auriculaire gauche tandis que le disque proximal (102) est configuré pour le côté auriculaire droit ; et dans lequel lesdits disques (101, 102) comprennent un matériau thrombogène
**caractérisé en ce que** le disque distal sans moyeu (101) incorpore une section centrale tissée (106) et ledit dispositif (D) comprend des fils non tissés (109) configurés sur un ou plusieurs étages, chaque étage étant intercalé avec une ou plusieurs tresses et tressé avec les fils provenant de la section centrale tissée (106) et des étages précédents pour former le dispositif (D).

2. Dispositif (D) selon la revendication 1, dans lequel une armure de la section centrale (106) est une armure simple ou une armure sergée.

3. Dispositif (D) selon la revendication 1, dans lequel les extrémités distales où se rencontrent les extrémités de fil des fils non tissés (109) sont combinées ensemble dans un moyeu incorporant un mécanisme de libération.
